(19)

Europäisches
Patentamt
European
Patent Office
Office européen
des brevets

(11) **EP 3 848 870 A1**

(12) **EUROPEAN PATENT APPLICATION**

(43) Date of publication:
**14.07.2021  Bulletin 2021/28**

(51) Int Cl.:
**G06Q 10/10** (2012.01)   **G06Q 50/22** (2018.01)
**G06Q 50/12** (2012.01)   **G16H 20/60** (2018.01)

(21) Application number: **20151353.8**

(22) Date of filing: **13.01.2020**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
**KH MA MD TN**

(71) Applicant: **Koninklijke Philips N.V.**
**5656 AG Eindhoven (NL)**

(72) Inventors:
• **KUI, Shirley**
**5656 AE Eindhoven (NL)**

• **JIN, Ya Fang**
**5656 AE Eindhoven (NL)**
• **MA, Henry**
**5656 AE Eindhoven (NL)**
• **XIAO, Weimin**
**5656 AE Eindhoven (NL)**
• **SUN, Winnie**
**5656 AE Eindhoven (NL)**

(74) Representative: **Philips Intellectual Property &
Standards
High Tech Campus 5
5656 AE Eindhoven (NL)**

(54) **NUTRITIONAL VALUE CALCULATION OF A DISH**

(57)     A computer-implemented method (100) of calculating a nutritional value of a dish is disclosed. The method comprises, with a processor (22) of said computer (20), identifying (103) the main ingredients of the dish; for each identified main ingredient, obtaining (105) an amount of said main ingredient in the dish and obtaining a nutritional value of said identified main ingredient from a main ingredient library (32); determining (109) a cooking method of the dish; identifying (111) additional ingredients of the dish based on the determined cooking method of the dish, said identifying including obtaining an amount of the additional ingredient in the dish and a nutritional value of the additional ingredient from an additional ingredient library (34); calculating (113) the nutritional value of the dish based on the obtained amounts of main ingredients and additional ingredients and their respective nutritional values; and generating (115) an output indicating the calculated nutritional value of the dish. Also disclosed are a computer program product for implementing this method and a computer system including this computer program product.

FIG. 1

**EP 3 848 870 A1**

**Description**

FIELD OF THE INVENTION

The present invention relates to a computer-implemented method of calculating a nutritional value of a dish.
The present invention further relates to a computer program product for implementing such a method on a computer.
The present invention still further relates to a computer system comprising such a computer program product.

BACKGROUND OF THE INVENTION

Modern society has seen a shift from more active to more sedentary lifestyles, with nutrition having shifted towards processed food products with high levels of saturated fats, sugars, salt and other ingredients that are potentially harmful to the consumer's health. This has led to a notable increase in lifestyle diseases such as type-2 diabetes, cardiovascular diseases, cancer, to name but a few. However, there is an increasing awareness of the potential risks of unhealthy eating habits, such that there has been a drive towards healthier eating. This has been aided by the proliferation of Internet-based services such as recipe databases, in which the recipes of many dishes are catalogued together with their nutritional values, such that users of such a database, e.g. through an app on a computer device such as a smart phone, tablet computer or the like, can maintain a healthy diet by selecting recipes based on such nutritional information.
Such a recipe database is typically built and expanded by users adding new recipes to the database. Nevertheless, for such a recipe database to be able to successfully assist its users in making informed choices about their diet, it is important that the database contains a large variety of recipes with accurate nutritional information. This therefore requires the users who add new recipes to such a database to provide accurate nutritional information for such new recipes. For example, a user may update such a database through the app interfacing with the recipe database by specifying the nutritional information of the respective ingredients of the recipe. This however is perceived as cumbersome and annoying by most users, which often leads to such nutritional information not being provided at all. In addition, most users do not have sufficient knowledge of nutrition to accurately provide the required nutritional information, which can lead to a recipe being tagged with inaccurate nutritional information, which is also undesirable.
South Korean patent application KR 2018/0116779 A discloses a method for evaluating the nutritional value of a dish by image and voice recognition with an artificial intelligence algorithm using a portable terminal including a camera, a control unit, a storage unit, a microphone, and a display unit. The method comprises the steps of: (a) photographing, by a camera, food on a menu before a meal; (b) receiving, by a control unit, a first image of the photographed food on the menu before a meal to recognize a type and amount of the food on the menu before a meal, and determining calories and nutrients thereof; (c) temporarily storing, by a storage unit, the recognized type and amount of the food on the menu before a meal and the determined calories and nutrients; (d) photographing, by the camera, the food on the menu after a meal; (e) receiving, by the control unit, a second image of the photographed food on the menu after a meal to calculate the difference between the second image and the temporarily stored type and amount of the food on the menu before a meal so as to estimate a type and amount of ingested food; and (f) receiving, by the control unit, data on the estimated type and amount of the ingested food to calculate the calories and nutrients so as to display the same on the display unit.
With this method, which relies on the availability of a recipe database to recognize the meal, the nutritional intake of a person eating the photographed meal may be estimated, as long as the recipe of the meal is present in the recipe database. However, this method cannot provide an accurate description of a recipe of the meal when not all ingredients are recognizable in such a photograph. Moreover, the amounts of such ingredients in the meal are unlikely to be accurately recognizable from such a photograph. In addition, the preparation method of the meal will have an impact on its nutritional value, which preparation method typically also cannot be clearly recognized from such a photograph. As such, this method is unsuitable to be used for updating a recipe database with a new recipe containing accurate nutritional information.

SUMMARY OF THE INVENTION

The present invention seeks to provide a computer-implemented method of calculating a nutritional value of a dish that can be used to update a recipe database with a new recipe containing accurate nutritional information.
The present invention further seeks to provide a computer program product for implementing such a method on a computer system.
The present invention still further seeks to provide a computer system comprising such a computer program product.

According to an aspect, there is provided a computer-implemented method of calculating a nutritional value of a dish, the method comprising, with a processor of said computer: identifying the main ingredients of the dish; for each main ingredient, obtaining an amount; and obtaining a nutritional value from a main ingredient library; determining a cooking method of the dish; identifying additional ingredients of the dish based on the determined cooking method of the dish, said identifying including obtaining an amount of the additional ingredient in the dish and a nutritional value of the additional ingredient from an additional ingredient library; calculating the nutritional value of the dish based on the obtained amounts of main ingredients and additional ingredients and their respective nutritional values; and generating an output indicating the calculated nutritional value of the dish.

With such a method, a user seeking to update a recipe database is not required to provide nutritional information, as this information instead is obtained through the computer-implemented method by accessing appropriate libraries to retrieve such information. Furthermore, by taking account of the cooking method of the dish, a particularly accurate estimation of its nutritional value can be obtained, as additional ingredients specific to such a cooking method are also included in the calculation of the nutritional value of the dish. Hence, a computer-implemented method is provided in which a user can update a recipe database with a new recipe for which an accurate estimate of its nutritional value is automatically generated, thereby greatly improving the user experience of updating a recipe database as well as improving the accuracy of the recipe database.

Identifying the main ingredients of the dish may comprise receiving a list of ingredients as a user input. The user may specify the recipe of the dish in terms of individual ingredients (and their amounts), which allows for a straightforward determination of the contribution of each ingredient to the overall nutritional value of the dish.

Alternatively, identifying the main ingredients of the dish may comprise receiving a name of the dish as a user input; and identifying said main ingredients from the received name of the dish. For example, the name of the dish may be indicative of the inclusion of at least some of its ingredients, such that these ingredients may be derived from the specified name of the dish. This further enhances the user experience with such a computer-implemented method. Such a user input may be provided in any suitable form, such as for example a manual text input, voice input (speech recognition), photo recognition of the dish or name of the dish, e.g. when photographing a description of the dish, and so on.

The computer-implemented method may include obtaining a short-hand name of at least one of said main ingredients; and identifying a full name of said at least one of said main ingredients in a synonyms library based on the obtained short-hand name of said at least one of said main ingredients. This may further enhance the simplicity of the user inputs to be provided, whilst at the same time maintaining the accuracy of the nutrient value calculation of the dish.

Determining the cooking method of the dish may comprise receiving the cooking method as a user input such as to increase the likelihood of an accurate determination of the cooking method of the dish. Alternatively, determining the cooking method of the dish may comprise deriving the cooking method from a received name of the dish, for example where the name of the dish is indicative of its cooking method.

In the computer-implemented method, obtaining an amount of the main ingredient in the dish may comprise calculating said amount as a fraction of the total weight of the dish. In an embodiment, calculating said amount as a fraction of the total weight of the dish is based on the formula $a_i = W_i/W$, in which $a_i$ is said fraction, $W_i$ is the weight of the ingredient in the dish and W is the total weight of the dish. This allows for a particularly accurate estimation of the nutritional value of the dish.

In a particularly advantageous embodiment, calculating the nutritional value of the dish comprises calculating at least one of the energy content of the dish, the fat content of the dish, the carbohydrate content of the dish and additional nutrient content of the dish such that several different types of nutritional value of the dish may be expressed, thereby enhancing the nutritional information associated with the recipe of the dish.

For example, calculating at least one of the energy content of the dish, the fat content of the dish, the carbohydrate content of the dish and additional nutrient content of the dish comprises using at least one of the following formulas:

$$Y_{energy} = \frac{W - X_{add}}{W} * \sum_{i=1}^{N} a_i * X_{i\,energy} + X_{oil} * A + \left(X_{sugar} + X_{starch}\right) * B$$

$$Y_{fat} = \frac{W - X_{add}}{W} * \sum_{i=1}^{N} a_i * X_{i\,fat} + X_{oil}$$

$$Y_{carb} = \frac{W - X_{add}}{W} * \sum_{i=1}^{N} a_i * X_{i\,carb} + \left(X_{sugar} + X_{starch}\right)$$

$$Y_{add} = \frac{W - X_{add}}{W} * \sum_{i=1}^{N} a_i * X_{i\,add}$$

in which $Y_{energy}$ is the energy content of the total weight W of the dish; $Y_{fat}$ is the fat content of the total weight W of the dish; $Y_{carb}$ is the carbohydrate content of the total weight W of the dish; $Y_{add}$ is the additional nutrient content of the total weight W of the dish; $a^i$ is a weight fraction of the $i^{th}$ main ingredient from N main ingredients of the dish based on the total weight of the dish, in which N is a positive integer; $X_{add}$ is an average combined weight of the additional ingredients in the total weight W of the dish; $X_{oil}$ is an average weight of the amount of oil used for preparing the total weight W of the dish; $X_{sugar}$ is an average weight of added sugar in the total weight W of the dish; $X_{starch}$ is an average weight of added starch in the total weight W of the dish; $X_{i\,energy}$ is a unit energy content of the $i^{th}$ main ingredient in the dish; $X_{i\,fat}$ is a unit fat content of the $i^{th}$ main ingredient in the dish; $X_{i\,carb}$ is a unit carbohydrate content of the $i^{th}$ main ingredient in the dish; $X_{i\,add}$ is a unit additional nutrient content of the $i^{th}$ main ingredient in the dish; and A and B are scaling factors.

The scaling factor A may be 9 and the scaling factor B may be 4, although other suitable values may be used instead.

In an embodiment, the computer-implemented method further comprises transmitting said generated output to a client device for informing a user of the client device of the calculated nutritional value of the dish. For example, the user may use the computer-implemented method to obtain the nutritional value of a dish in this manner, e.g. to determine whether the dish fits within a particular diet or nutritional plan.

In another embodiment, the computer-implemented method further comprises transmitting said generated output to a nutritional value database for storing the calculated nutritional values of said dish in said database. As previously explained, such a nutritional value database may be a recipe database to which the recipe of the dish is added together with its calculated nutritional value such that other users may retrieve this information from the database at a later stage.

In accordance with another aspect, there is provided a computer program product computer program product comprising a computer readable storage medium having computer readable program instructions embodied therewith for, when executed on a processor of a computer system, cause the processor to implement the method of any of the herein described embodiments. Such a computer program product may be used to configure a computer system such as a personal computer, portable computer such as a smart phone or tablet computer, internet server or the like to calculate the nutritional value of a dish not present in a recipe database, such that a user of such a computer system may retrieve such nutritional information by running the computer readable program instructions on the processor of the computer system, e.g. to obtain nutritional information about such a dish or to update a nutritional database such as a recipe database with such information as previously explained.

In accordance with yet another aspect, there is provided a computer system comprising a processor and the computer program product of any of the herein described embodiments, wherein the processor is arranged to execute the computer readable program instructions embodied by said computer program product to allow the user of such a computer system to retrieve such nutritional information.

BRIEF DESCRIPTION OF THE DRAWINGS

Embodiments of the invention are described in more detail and by way of non-limiting examples with reference to the accompanying drawings, wherein:

FIG. 1 schematically depicts a computer system according to an embodiment; and
FIG. 2 is a flowchart of a computer-implemented method according to an embodiment.

DETAILED DESCRIPTION OF THE EMBODIMENTS

It should be understood that the Figures are merely schematic and are not drawn to scale. It should also be understood that the same reference numerals are used throughout the Figures to indicate the same or similar parts.

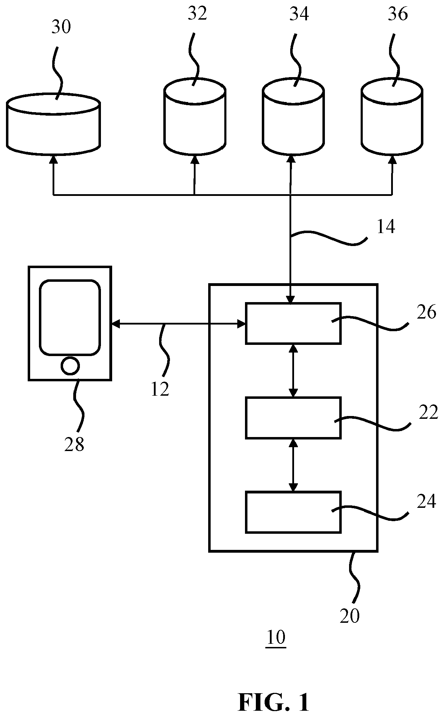

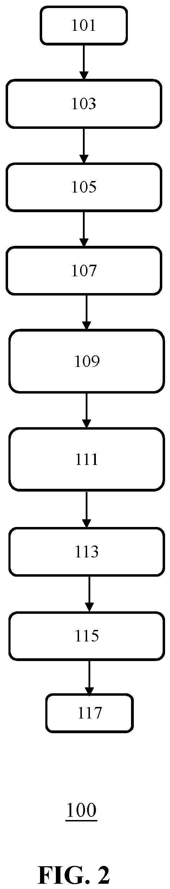

FIG. 1 schematically depicts an example infrastructure 10 including a computer system 20 according to an example embodiment of the present invention. The computer system 20 is arranged to implement a method 100 according to the present invention, an example embodiment of which is depicted by the flowchart in FIG. 2. The computer system 20 typically comprises a processor 22. In the context of the present invention, the processor 22 may take any suitable form, e.g. a single processor having one or more processing cores or a group of processors arranged to cooperate with each other such as to behave like a single processor. Other suitable implementations of such a processor 22 will be immediately apparent to the skilled person. The processor 22 has access to a computer readable storage medium 24. The computer readable storage medium 24 may take any suitable shape, such as a memory, solid state disk, hard disk or the like when integrated in the computer system 20. Alternatively, the processor 22 may have access to a remote computer readable storage medium 24 over a network connection such as a local area network, Internet, or the like. Such a remote computer readable storage medium 24 may take any suitable shape, such as a network attached storage (NAS), a storage area network (SAN), cloud storage or the like. The computer readable storage medium 24 typically contains computer readable program instructions for execution by the processor 22 of the computer system 20. These computer readable program instructions cause the processor 22 to implement any of the herein described embodiments of the method 100.

The computer system 20 further comprises one or more data communication interfaces 26. The one or more data communication interfaces 26 are communicatively coupled to the processor 22 such that the processor 22 may receive data from remote devices or transmit data to such a remote devices using the one or more data communication interfaces 26. The one or more data communication interfaces 26 may take any suitable form, such as a Bluetooth communication module, Wi-Fi communication module, an Ethernet card, a mobile communication standard interface such as a 4G or 5G communication module, and so on. Many more examples of suitable data communication or network interfaces will be immediately apparent to the skilled person. For example, the computer system 20 may be adapted to provide a service such as an Internet-based service in which a client device 28 may be connected to the computer system 20 over a data connection 12 to access such a service. The client device 28 may take any suitable shape, such as a personal computer, a portable electronic device such as a smart phone or tablet computer, a laptop computer and so on. The data connection 12 may be a P2P connection such as Bluetooth connection, a network connection such as a (wireless) Internet connection, a mobile communication standard connection, and so on. The computer system 20 further has access to a nutritional database 30 and a number of libraries 32, 34, 36 over at least one further data connection 14, e.g. a network connection such as an Internet connection or the like.

It should be understood that although the nutritional database 30 and the libraries 32, 34 and 36 are shown as separate entities, this is by way of illustration only, as at least some of these entities may form part of a single entity, e. g. a single data structure or the like. Similarly, although the computer system 20 is shown as a separate entity to the client device 28, in alternative embodiments the client device 28 comprises at least part of the computer system 20 such that the client device 28 may directly liaise with the nutritional database 30 and the libraries 32, 34 and 36. The computer system 20 may take any suitable form, such as a server providing Internet services, a personal computer, a portable computer or electronic device, and so on.

The aforementioned infrastructure may be used to implement embodiments of the method 100 of the present invention, which will now be described in further detail with the aid of FIG. 2. The method 100 starts in operation 101, which may involve establishing a connection between the client device 28 and the computer system 20 over the data connection 12, e.g. to access a nutritional database 30 such as a recipe database through the computer system 20, or to directly access the nutritional database 30 with the client device 28 in case the client device 28 comprises the computer system 20. This may further involve launching a software program such as an app on the client device 28 through which the user of the client device 28 can provide relevant information to the computer system 20, e.g. an Internet-based service for updating the nutritional database 30.

In operation 103, the processor 22 of the computer system 20 receives information from the client device 28 regarding the ingredients of a dish or recipe to be added to the nutritional database 30, or for which nutritional information is to be returned to the client device 28. For example, the user of the client device 28 may provide the processor 22 with a list of main ingredients of the dish through the software program running on the client device 28. This for instance may be achieved by the user by selection of ingredients from a list of ingredients presented to the user in any suitable manner on the client device 28 by the execution of the software program thereon. Preferably, for the N ingredients specified in this manner, with N being a positive integer, the user further specifies the amount of each main ingredient in the dish, e.g. in grams (g) or another suitable unit of weight, or in millilitres (ml) or another suitable unit of volume.

The processor 22 may convert a specified volume of a particular main ingredient into an equivalent weight, e. g. using a reference density such as 1g/ml for the volume such that all main ingredient amounts are expressed in the same type of unit, e.g. a unit of weight. The weight of an $i^{th}$ main ingredient of the N ingredients may be expressed as $W_i$. If the amounts of the main ingredients are not specified by the user, the processor 22 may assume that each main ingredient is present in the same amount, i.e. for N main ingredients and a total weight W of the dish, each main ingredient is present in an amount W/N. The total weight W of the dish may be specified by the user. Alternatively, where the infrastructure 10 includes scales or the like communicatively coupled to the computer system 20, the total weight W of the dish when prepared, e.g. by the user, may be established by weighing the prepared dish with such scales. Such scales for instance may be a standalone device or may be integrated in a kitchen appliance such as food processing apparatus for preparing the dish.

Alternatively, the user of the client device 28 may provide the processor 22 with the name of the dish, from which the processor 22 derives the main ingredients of the dish. Such a user input may be provided in any suitable form, such as for example a manual text input, voice input (speech recognition), photo recognition of the dish or name of the dish, e.g. when photographing a description of the dish, and so on. For example, the user of the client device 28 may specify that the name of the dish is 'chicken teriyaki', from which the processor 22 can deduct that one of the main ingredients is chicken, and can consult the nutritional database 30 or another suitable database or library, e.g. over the further data connection 14, to find other dishes named 'teriyaki' to establish the main ingredients typically present in dishes with that name. The processor 22 may obtain the respective amounts of the main ingredients of the named dish by copying the amounts of the main ingredients found in existing recipes, e.g. 'teriyaki' recipes in the given example, within the nutritional database 30 or another suitable database, or in the absence of such information, assume that each main ingredient is present in the same amount, i.e. for N main ingredients and a total weight W of the dish, each main ingredient is present in an amount W/N. The total weight W may be established as previously explained.

In the foregoing scenarios, if an ingredient is specified in short-hand form, e.g. egg for chicken egg, ham for pork ham and so on, the processor 22 may further consult a synonyms library 36 to obtain the full name of such a specified ingredient. This for instance may be achieved by checking for each specified ingredient if a synonym (i.e. full name) exists in the synonyms library 36. The synonyms library 36 typically contains the short name or synonym of main ingredients that can be found in the main ingredient library 32, such that upon finding the full name of such a main ingredient in the synonyms library 36, the nutritional information of such a main ingredient can be retrieved from the main ingredient library 32 based on the identified full name.

In operation 105, the processor 22 accesses the main ingredient library 32 to obtain the nutritional value of each identified main ingredient. In the context of the present application, where reference is made to the nutritional value of an ingredient, this may include one or more of the energy content, fat content, carbohydrate content such as sugar content, and additional nutrient content of such an ingredient. The additional nutrient content may be one or more of protein content, vitamin content, e.g. vitamin A, B1, B6, B12, C, D content and so on, trace element content such as zinc, iron, calcium content, and so on. Such contents may be expressed in any suitable manner, such as (milli-) grams per unit weight, e.g. 1 gram, 100 grams or the like, of the main ingredient. Many other suitable expressions will be immediately apparent to the skilled person. The main ingredient library 32 may form part of a food composition database or the like in some embodiments. The main ingredients library 32 typically contains all ingredients that can be used in the preparation of edible foods and their nutritional values. Consequently, the main ingredients library may contain up to 2,000 or more different ingredients, including fruits, vegetables, meats, poultry, seafood such as fish and shellfish, protein sources such as types of egg, and so on. In this manner, the recipe of virtually any dish can be expressed in terms of main ingredients stored in the main ingredients library, such that the nutritional value of virtually any dish can be accurately estimated using the information in this library.

In operation 107, the weight fractions ai = Wi/W may be calculated by the processor 22 for each $i^{th}$ main ingredient based on the obtained weight for each main ingredient and the obtained total weight of the dish. The operation 107 may be performed at any suitable point in time, e.g. before, after or at the same time as operation 105.

In operation 109, the processor 22 determines the cooking method of the dish, e.g. stir-frying, boiling, (oven-) baking, steaming, and so on. The cooking method of the dish may be determined by the processor 22 from a user input provided with the client device 28, or may be derived from the name of the dish, e.g. a 'chicken casserole' may refer to a dish that has been oven baked, and so on.

In operation 111, the processor 22 accesses the additional ingredients library 34 to retrieve the additional ingredients and their amounts based on the specified cooking method of the dish. The additional ingredients library 34 typically contains additional ingredient information specific to each particular cooking method, e.g. amounts of oil and seasoning used per unit weight of the dish for a particular cooking method. This is an important feature of the embodiments of the present invention, as the cooking method of a dish can have a marked impact on its nutritional value, such that including the cooking method in the calculation of the nutritional value of a dish greatly improves the accuracy of the estimation of its nutritional value.

At this point, the processor 22 has gathered all information required to calculate the nutritional values of the dish specified by the user, i.e. the nutritional information of the main ingredients, the amounts of the main ingredients, and the nutritional information and amounts of the additional ingredients as a function of cooking method, such that the processor 22 can proceed to calculating the nutritional value(s) of the dish in operation 113. In an example embodiment, the processor 22 is adapted to calculate these nutritional values using the following algorithms or formulas:

$$Y_{energy} = \frac{W - X_{add}}{W} * \sum_{i=1}^{N} a_i * X_{i\,energy} + X_{oil} * A + \left(X_{sugar} + X_{starch}\right) * B$$

$$Y_{fat} = \frac{W - X_{add}}{W} * \sum_{i=1}^{N} a_i * X_{i\,fat} + X_{oil}$$

$$Y_{carb} = \frac{W - X_{add}}{W} * \sum_{i=1}^{i} a_i * X_{i\,carb} + (X_{sugar} + X_{starch})$$

$$Y_{add} = \frac{W - X_{add}}{W} * \sum_{i=1}^{N} a_i * X_{i\,add}$$

in which:

N is the number of main ingredients in the dish;

$Y_{energy}$ is the energy content of the total weight W of the dish;

$Y_{fat}$ is the fat content of the total weight W of the dish;

$Y_{carb}$ is the carbohydrate content of the total weight W of the dish;

$Y_{add}$ is the additional nutrient content of the total weight W of the dish;

$a^i$ is a weight fraction of the $i^{th}$ main ingredient of the dish based on the total weight of the dish;

$X_{add}$ is an average combined weight of the additional ingredients in the total weight W of the dish in the specified cooking method as retrieved from additional ingredients library 34;

$X_{oil}$ is an average weight of the amount of oil used for preparing the total weight W of the dish in the specified cooking method as retrieved from additional ingredients library 34;

$X_{sugar}$ is an average weight of added sugar in the total weight W of the dish in the specified cooking method as retrieved from additional ingredients library 34;

$X_{starch}$ is an average weight of added starch in the total weight W of the dish in the specified cooking method as retrieved from additional ingredients library 34;

$X_{i\,energy}$ is a unit energy content of the $i^{th}$ main ingredient in the dish as retrieved from the main ingredients library 32;

$X_{i\,fat}$ is a unit fat content of the $i^{th}$ main ingredient in the dish as retrieved from the main ingredients library 32;

$X_{i\,carb}$ is a unit carbohydrate content of the $i^{th}$ main ingredient in the dish as retrieved from the main ingredients library 32;

$X_{i\,add}$ is a unit additional nutrient content of the $i^{th}$ main ingredient in the dish as retrieved from the main ingredients library 32; and

A and B are scaling factors.

The unit content variables, $X_{i\,fat}$, $X_{i\,carb}$ and $X_{i\,add}$ typically are dimensionless variables that for example may be expressed as a weight of the particular nutritional element (e.g. fat, carbohydrate, additional nutrient) per unit amount of total weight, e.g. 100g, of the dish. The scaling factors A and B may be chosen such as to accurately reflect the energy density of the cooking oil (scaling factor A) and added sugar and starch (scaling factor B) included in the dish as a function of its cooking method. In example embodiments, A is about 9 and B is about 4. The added starch content $X_{starch}$ may reflect the amount of starch released by a starchy food such as beans or corn added to the dish, e.g. as a binding agent for a sauce, soup or the like.

Upon calculation of the nutritional values of the dish or recipe, the processor 22 proceeds to operation 115 in which the processor 22 generates an output indicating the calculated one or more nutritional values of the dish or recipe, e.g. one or more of $Y_{energy}$, $X_{i\,fat}$, $X_{i\,carb}$ and $X_{i\,add}$. Preferably, the output includes all of $Y_{energy}$, $X_{i\,fat}$, $X_{i\,carb}$ and $X_{i\,add}$. The output generated by the processor 22 may be transmitted by the data communication interface 26 to the client device 28, such as to inform the user of the client device 28 of the nutritional values of the dish input by the user into the computer system 20 for calculation of its nutritional values. Alternatively or additionally, the output generated by the processor 22 may be transmitted by the data communication interface 26 to the nutritional database 30 for storage of the dish recipe and its calculated nutritional values therein, such that the recipe and its nutritional values becomes available to other users accessing the nutritional database 30, e.g. a recipe database, allowing such other users to select the recipe from this database for preparation of the dish, whereby such other users are provided with accurate nutritional information of the dish as calculated in accordance with the above described method 100. Upon generation of this output signal, the method 100 terminates in operation 117.

The above described embodiments of the method 100 may be realized by computer readable program instructions embodied on a computer readable storage medium having, when executed on the processor 22, cause the processor to implement the method 100. Any suitable computer readable storage medium may be used for this purpose, such as for example an optically readable medium such as a CD, DVD or Blu-Ray disc, a magnetically readable medium such as a hard disk, an electronic data storage device such as a memory stick or the like, and so on. The computer readable storage medium may be a medium that is accessible over a network such as the Internet, such that the computer readable program instructions may be accessed over the network. For example, the computer readable storage medium may be a network-attached storage device, a storage area network, cloud storage or the like. The computer readable storage medium may be an Internet-accessible service from which the computer readable program instructions may be obtained. In an embodiment, the computer system 20 is adapted to retrieve the computer readable program instructions from such a computer readable storage medium and to create a new computer readable storage medium by storing the retrieved computer readable program instructions in a data storage arrangement (not shown), e.g. in a memory device or the like forming part of the data storage arrangement.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention can be implemented by means of hardware comprising several distinct elements. In the device claim enumerating several means, several of these means can be embodied by one and the same item of hardware. The mere fact that certain measures are recited in mutually different dependent claims does not indicate that a combination of these measures cannot be used to advantage.

**Claims**

1. A computer-implemented method (100) of calculating a nutritional value of a dish, the method comprising, with a processor (22) of said computer (20):

   identifying (103) the main ingredients of the dish;
   for each identified main ingredient, obtaining (105) an amount of said main ingredient in the dish and obtaining a nutritional value of said identified main ingredient from a main ingredient library (32);
   determining (109) a cooking method of the dish;
   identifying (111) additional ingredients of the dish based on the determined cooking method of the dish, said identifying including obtaining an amount of the additional ingredient in the dish and a nutritional value of the additional ingredient from an additional ingredient library (34);
   calculating (113) the nutritional value of the dish based on the obtained amounts of main ingredients and additional ingredients and their respective nutritional values; and
   generating (115) an output indicating the calculated nutritional value of the dish.

2. The computer-implemented method (100) of claim 1, wherein identifying (103) the main ingredients of the dish comprises receiving a list of ingredients and optionally an amount of each main ingredient in said list as a user input.

3. The computer-implemented method (100) of claim 1, wherein identifying (103) the main ingredients of the dish comprises:

   receiving a name of the dish as a user input; and
   identifying said main ingredients from the received name of the dish.

4. The computer-implemented method (100) of claim 2 or 3, further comprising:

   obtaining a short-hand name of at least one of said main ingredients; and
   identifying a full name of said at least one of said main ingredients in a synonyms library (36) based on the obtained short-hand name of said at least one of said main ingredients.

5. The computer-implemented method (100) of any of claims 1-4, wherein determining (109) the cooking method of the dish comprises receiving the cooking method as a user input.

6. The computer-implemented method (100) of any of claims 1-4, wherein determining (109) the cooking method of the dish comprises deriving the cooking method from a received name of the dish.

7. The computer-implemented method (100) of any of claims 1-6, wherein obtaining (105) an amount of the main ingredient in the dish comprises calculating (107) said amount as a fraction of the total weight of the dish.

8. The computer-implemented method (100) of claim 7, wherein calculating (107) said amount as a fraction of the total weight of the dish is based on the formula $a_i = W_i/W$, in which $a_i$ is said fraction, $W_i$ is the weight of the ingredient in the dish and W is the total weight of the dish.

9. The computer-implemented method (100) of any of claims 1-8, wherein calculating (113) the nutritional value of the dish comprises calculating at least one of the energy content of the dish, the fat content of the dish, the carbohydrate content of the dish and additional nutrient content of the dish.

10. The computer-implemented method (100) of claim 9, wherein calculating at least one of the energy content of the dish, the fat content of the dish, the carbohydrate content of the dish and additional nutrient content of the dish comprises using at least one of the following formulas:

$$Y_{energy} = \frac{W - X_{add}}{W} * \sum_{i=1}^{N} a_i * X_{i\ energy} + X_{oil} * A + \left(X_{sugar} + X_{starch}\right) * B$$

$$Y_{fat} = \frac{W - X_{add}}{W} * \sum_{i=1}^{N} a_i * X_{i\ fat} + X_{oil}$$

$$Y_{carb} = \frac{W - X_{add}}{W} * \sum_{i=1}^{i} a_i * X_{i\ carb} + \left(X_{sugar} + X_{starch}\right)$$

$$Y_{add} = \frac{W - X_{add}}{W} * \sum_{i=1}^{N} a_i * X_{i\ add}$$

in which:

$Y_{energy}$ is the energy content of the total weight W of the dish;
$Y_{fat}$ is the fat content of the total weight W of the dish;
$Y_{carb}$ is the carbohydrate content of the total weight W of the dish;
$Y_{add}$ is the additional nutrient content of the total weight W of the dish;
$a^i$ is a weight fraction of the $i^{th}$ main ingredient of the dish based on the total weight of the dish;
$X_{add}$ is an average combined weight of the additional ingredients in the total weight W of the dish;
$X_{oil}$ is an average weight of the amount of oil used for preparing the total weight W of the dish;
$X_{sugar}$ is an average weight of added sugar in the total weight W of the dish;
$X_{starch}$ is an average weight of added starch in the total weight W of the dish;
$X_{i\ energy}$ is a unit energy content of the $i^{th}$ main ingredient in the dish;
$X_{i\ fat}$ is a unit fat content of the $i^{th}$ main ingredient in the dish;
$X_{i\ carb}$ is a unit carbohydrate content of the $i^{th}$ main ingredient in the dish;
$X_{i\ add}$ is a unit additional nutrient content of the $i^{th}$ main ingredient in the dish; and
A and B are scaling factors.

11. The computer-implemented method (100) of claim 10, wherein A is 9 and B is 4.

12. The computer-implemented method (100) of any of claims 1-11, further comprising transmitting said generated output to a client device (28) for informing a user of the client device of the calculated nutritional value of the dish.

13. The computer-implemented method (100) of any of claims 1-12, further comprising transmitting said generated output to a nutritional value database (30) for storing the calculated nutritional values of said dish in said database.

14. A computer program product comprising a computer readable storage medium (24) having computer readable program instructions embodied therewith for, when executed on a processor (22) of a computer system (20), cause the processor to implement the method of any of claims 1-13.

15. A computer system (20) comprising a processor (22) and the computer program product of claim 14, wherein the processor is arranged to execute the computer readable program instructions embodied by said computer program product.

**FIG. 1**

100

FIG. 2

Europäisches
Patentamt

European
Patent Office

Office européen
des brevets

# EUROPEAN SEARCH REPORT

Application Number

EP 20 15 1353

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/031995 A1 (BHATT DHRUV A [US] ET AL) 2 February 2017 (2017-02-02) * figures 1-2,4-6 * ----- | 1-15 | INV. G06Q10/10 G06Q50/22 G06Q50/12 G16H20/60 |
| X | WO 2017/075498 A1 (FORQ INC [US]) 4 May 2017 (2017-05-04) * figures 7-12 * ----- | 1-15 | |
| A | US 2019/130786 A1 (KUMBAKONAM SUNDARESAN NATARAJAN [IN]) 2 May 2019 (2019-05-02) * figures 1-3 * ----- | 1-15 | |

TECHNICAL FIELDS
SEARCHED        (IPC)

G06Q
G16H

The present search report has been drawn up for all claims

| Place of search | Date of completion of the search | Examiner |
|---|---|---|
| Munich | 11 May 2020 | Heselius, Per |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 20 15 1353

This annex lists the patent family members relating to the patent documents cited in the above-mentioned European search report.
The members are as contained in the European Patent Office EDP file on
The European Patent Office is in no way liable for these particulars which are merely given for the purpose of information.

11-05-2020

| Patent document cited in search report | | Publication date | Patent family member(s) | | Publication date |
|---|---|---|---|---|---|
| US 2017031995 | A1 | 02-02-2017 | US | 2017031966 A1 | 02-02-2017 |
| | | | US | 2017031995 A1 | 02-02-2017 |
| | | | US | 2018004789 A1 | 04-01-2018 |
| | | | US | 2018004790 A1 | 04-01-2018 |
| WO 2017075498 | A1 | 04-05-2017 | EP | 3369064 A1 | 05-09-2018 |
| | | | US | 2018308143 A1 | 25-10-2018 |
| | | | WO | 2017075498 A1 | 04-05-2017 |
| US 2019130786 | A1 | 02-05-2019 | US | 2019130786 A1 | 02-05-2019 |
| | | | WO | 2019083853 A1 | 02-05-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20180116779 A